# EUROPÄISCHE PATENTSCHRIFT

(11) **EP 1 083 196 B1**
(45) Veröffentlichungstag und Bekanntmachung des Hinweises auf die Patenterteilung: **14.09.2005**
(21) Anmeldenummer: 00117131.3
(22) Anmeldetag: 10.08.2000
(51) Int. Cl.: C08J 3/12, C08J 3/205, B29B 9/06, A61K 9/20, A61J 3/10

(54) **Unterwassergranulation wirkstoffhaltiger Schmelzen**
Underwater granulation of therapeutic agent containing melts
Granulation sous l'eau à l'état fondu comprenant un principe actif

(30) Priorität: 10.09.1999 DE 19943501
(43) Veröffentlichungstag der Anmeldung: 14.03.2001
(73) Patentinhaber: BASF Aktiengesellschaft, 67056 Ludwigshafen (DE)
(72) Erfinder: Ernst, Andreas, Dr., 67551 Worms (DE); Kessler, Thomas, 67105 Schifferstadt (DE); Berndl, Gunther, Dr., 67273 Herxheim (DE)

(56) Entgegenhaltungen:
- DE-A- 19 809 242
- US-A- 4 150 595
- US-A- 4 801 460
- US-A- 4 880 585
- US-A- 5 143 673
- DATABASE WPI Section Ch, Week 199517 Derwent Publications Ltd., London, GB; Class A23, AN 1995-125824 XP002156052 & JP 07 047545 A (MITSUBISHI KASEI CORP), 21. Februar 1995 (1995-02-21)

## Beschreibung

Die vorliegende Erfindung betrifft ein Verfahren zur Herstellung von wirkstoffhaltigen Granulaten auf Basis thermoplastisch verarbeitbarer Polymere durch Schmelzextrusion und Unterwassergranulation.

Unterwassergranulation oder Unterwasserpelletierung ist als effektive Granulationsmethode für thermoplastische Materialien zur industriellen Herstellung von Plastikgranulaten allgemein bekannt.

Entsprechende Vorrichtungen und Verfahren sind beispielsweise in der DE-A 2646309, der US-A 4264553 oder der US-A 5143673 beschrieben. Hierbei wird die heiße plastische Schmelze aus einem Extruder durch eine Lochscheibe direkt in ein flüssiges Kühlmedium, in diesem Fall Wasser, gefördert. Die austretenden Plastikstränge werden durch eine an der Lochscheibe angebrachten Schneidvorrichtung in Granulate zerteilt und durch das meist im Kreislauf gefahrene Wasser wegtransportiert, abgetrennt und getrocknet. Durch diesen, flüssigkeitsgekühlten Prozeß ist es möglich sehr kleine Partikelgrößen einheitlich, kontinuierlich und im technischen Maßstab bis herunter zum Submillimeterbereich zu produzieren. Durch die Verwendung von Kühlmedien mit einer hohen Wärmekapazität und einem hohen Wärmeübergang wie Wasser, werden die noch plastischen Granulate schnell abgekühlt, verkleben nicht und lassen sich in Form und Größe sehr einheitlich herstellen.

Demgegenüber ist die Verwendung eines sogenannten Heißabschlages mit Luftkühlung zur Granulierung der extrudierten Stränge aufgrund der langsamen und schlechten Wärmeabführung deutlich schwieriger.

Wirkstoffhaltige Formulierungen, die beispielsweise als Wirkstoff Vitamine enthalten, werden zur Gesunderhaltung von Mensch und Tier parallel zur Nahrungsaufnahme verabreicht oder der Nahrung als Additiv zugesetzt. Ein großer Anteil der hergestellten Formulierungen von Vitaminen, Vitaminoiden oder anderen Nahrungsergänzungsmitteln wird für die Tierernährung benötigt. Da die dort verwendeten Futtermittel als Mahlgut mit einer mittleren Teilchengröße von 0.3-0.5mm verabreicht werden, sollten zugesetzte Futtermitteladditive - um Entmischungen zu vermeiden - etwa die gleiche Größe und Einheitlichkeit aufweisen. Auch bei der Herstellung von Granulaten für Arzneimittel ist eine einheitliche Korngröße von besonderer Bedeutung, da das Auflösungsverhalten und damit die Bioverfügbarkeit von der Korngröße abhängen. Die bisher verwendeten Arzneimittelgranulate, Nahrungsergänzungsmittel oder Futtermitteladditive werden demzufolge meist in aufwendigen und teuren Mahl-, Granulier- und Sprühverfahren hergestellt.

Wirkstoffhaltige Zubereitungen, die durch Schmelzextrusion hergestellt werden, sind allgemein bekannt. Das Extrudieren von wirkstoffhaltigen Schmelzen wasserlöslicher Polymere, vorzugsweise von Copolymeren des Vinylpyrrolidons, ist beispielsweise aus der EP-A 240904 und der EP-A 240906 bekannt. In der EP-A 240 906 ist zudem die Schmelzextrusion von wirkstoffhaltigen Mischungen, die Copolymerisate von Methylmethacrylat und Acrylsäure, Copolymerisate von Vinylacetat und Crotonsäure als auch Ethylen/Vinylacetat-Copolymerisate beschrieben. Die Formgebung erfolgt über Spritzguß oder Extrusion mit anschließender Verformung des noch plastischen Stranges, z.B. durch Heißabschlag zu Granulat oder Verformen zu Tabletten. Die Formgebung erfolgt in allen genannten Beispielen an der Luft. Die so erzeugten pharmazeutischen Formen sind im allgemeinen wasserlöslich.

So beschreibt zum Beispiel auch die DE-A 19536387 die Schmelzextrusion und Formgebung vitaminhaltiger Produkte. Als Matrix werden wasserlösliche, thermoplastische Hydroxypropylcellulosen verwendet. In den Beispielen werden unter anderem Schmelzen aus Vitamin C oder β-Carotin zusammen mit Hydroxypropylcellulose durch Formkalander zu Tabletten gepresst. Zudem wird der Heißabschlag der wasserlöslichen Matrix zu Pellets erwähnt.

Zwar kann man solche wasserlöslichen Formulierungen mit Hilfe der Schmelzextrusion und anschließender Granulierung an der Luft herzustellen. Nachteilig ist aber, daß man auf diese Art häufig aufgrund der schlechten Wärmeabführung nicht die erforderliche (kleine) Teilchengröße, Einheitlichkeit und großtechnische Realisierbarkeit erreichen kann.

Aufgabe der Erfindung war es, ein kostengünstiges und einfaches Herstellverfahren für wirkstoffhaltige Formulierungen wie beispielsweise Arzneimittel oder Futtermitteladditive, die sich in Wasser bzw. im Magen-Darm-Trakt lösen oder selbst dispergieren, zu entwickeln.

Überraschenderweise konnte gezeigt werden, daß die Aufgabe gelöst werden kann, wenn man wirkstoffhaltige Mischungen mit Polymeren schmelzextrudiert, die in einem gewissen pH-Bereich in Wasser unlöslich sind und in einem anderen pH-Bereich in Wasser löslich sind.

Demgemäß wurde ein Verfahren zur Herstellung von Granulaten, die biologisch aktive Substanzen enthalten, welche für die Herstellung von Arzneimitteln, Futtermitteln, Nahrungsergänzungsmitteln und dietätischen 3 Mitteln verwendet werden, in denen die biologisch aktiven Substanzen homogen dispergiert in einer Matrix auf Basis mindestens eines thermoplastisch verarbeitbaren Polymeren, welches in wässrigem Medium eine pH-Wert abhängige Löslichkeit aufweist, vorliegen, durch homogenes Vermischen der Einsatzstoffe in der Schmelze und anschliessender Extrusion und Formgebung, gefunden, welches dadurch gekennzeichnet ist, daß, die Formgebung in einem Kühlmedium erfolgt, in dem die Granulate nicht löslich oder dispergierbar sind, wobei als Kühlmedium Wasser eingesetzt wird, dessen pH-Wert so eingestellt ist, daß die Granulate sich nicht lösen oder dispergierbar sind.

Zur Durchführung des erfindungsgemäßen Verfahrens werden die Einsatzstoffe zu einer homogenen Schmelze vermischt. Dies kann beispielsweise erfolgen, indem man ein physikalische Vormischung fester Einsatzstoffe in einen geeigneten Extruder oder Kneter einbringt, die Mischung unter Einsatz von mechanischer und thermischer Energie aufschmilzt, und die heiße, noch plastische Schmelze durch eine Lochscheibe oder eine Düsenplatte direkt in ein flüssiges Kühlmedium fördert. Enthält die Formulierung auch flüssige Inhaltsstoffe, so empfiehlt es sich, diese getrennt über eine Dosierpumpe zuzugeben. Verwendet man thermisch labile Wirkstoffe, so kann es sich auch empfehlen, zunächst eine Schmelze der thermoplastisch verarbeitbaren Polymere und gewünschtenfalls weiterer Formulierungshilfsstoffe herzustellen, und dann erst den Wirkstoff zuzugeben. Das Aufschmelzen erfolgt vorzugsweise in einer Schneckenmaschine, insbesondere in einem Doppelschneckenextruder, der vorzugsweise gleichsinnig drehend ist. Das erfindungsgemäße Verfahren erfolgt vorzugsweise in Abwesenheit von Lösungsmitteln. Im Falle, daß es erforderlich sein sollte, einen oder mehreren der Einsatzstoffe in Form einer Lösung zu der Mischung zu geben, können die dabei verwendeten Lösungsmittel in den hinteren Extruderzonen durch Anlegen eines Vakuums entfernt werden. Die extrudierte, noch thermoplastische Masse enthält dann kein Lösungsmittel mehr.

Das Aufschmelzen der Einsatzstoffe kann je nach Zusammensetzung der Mischung bei Temperaturen von 50 bis 300°C, vorzugsweise 70 bis 250°C, erfolgen.

Die austretenden plastischen Stränge werden durch eine an der Lochscheibe oder Düsenplatte angebrachten Schneidvorrichtung in Granulate zerteilt und durch das vorzugsweise im Kreislauf gefahrene Kühlmedium wegtransportiert, abgetrennt und getrocknet. Als Kühlmittel eignen sich beispielsweise flüssige Kohlenwasserstoffe wie Paraffine oder aromatische Kohlenwasserstoffe, oder vorzugsweise Wasser. Setzt man Wasser als Kühlmedium ein, so wird erfindungsgemäß der pH-Wert des Wassers mit Hilfe von Säuren oder Basen so eingestellt, daß sich die wirkstoffhaltige Matrix darin nicht löst oder selbst dispergiert. Erfindungsgemäß soll dies bedeuten, daß sich nicht mehr als 1 g/l der Granulate in Wasser lösen oder dispergieren lassen.

Durch die Verwendung von Kühlmedien mit einer hohen Wärmekapazität und einem hohen Wärmeübergang wie Wasser, werden die noch plastischen Granulate schnell abgekühlt, verkleben nicht und lassen sich in Form und Größe sehr einheitlich herstellen. Bevorzugt werden Granulate mit mittleren Teilchengrößen von 0,1 bis 5, vorzugsweise von 0,3 bis 3 mm hergestellt.

Das erfindungsgemäße Verfahren eignet sich prinzipiell zur Herstellung von biologisch aktive Substanzen enthaltenden Granulaten, in denen eine oder mehrere biologisch aktive Substanzen homogen in einer Matrix auf Basis thermoplastisch verarbeitbarer Polymere dispergiert ist. Homogen dispergiert kann erfindungsgemäß auch bedeuten, daß die biologisch aktiven Substanzen molekulardispers in der Matrix vorliegen, also sogenannte "feste Lösungen" darstellen.

Als biologisch aktive Substanzen kommen generell alle Substanzen in Betracht, die im Magen-Darm-Trakt von Mensch und Tier freigesetzt werden sollen. Dies können beispielsweise Pharmawirkstoffe, Vitamine, Vitaminoide, Carotinoide, Enzyme, Hormone, Aminosäuren oder sogenannte "Nutraceuticals", also Nahrungsergänzungsmittel und dietätische Mittel, sein.

Zudem ist es möglich auch Waschmittelinhaltsstoffe, Geruchs- und Geschmacksstoffe oder andere Aktivsubstanzen in der oben beschriebenen Weise zu formulieren.

Das erfindungsgemäße Verfahren und die erfindungsgemäße Vorrichtung eigenen sich zur Herstellung von partikulären Zubereitungen biologischer Substanzen. Biologisch aktive Substanzen sind erfindungsgemäß Stoffe, die in lebenden Organismen eine biologische Wirkung hervorrufen.

Das erfindungsgemäße Verfahren eignet sich beispielsweise zur Formulierung folgender Stoffe oder deren physiologisch akzeptablen Salzen, wobei die Salze auch in situ im Extruder erzeugt werden können:
- Antiinfektiva
   Aciclovir, Aminoglykoside, Amphotericin B, Azol-Antimykotika, Clotrimazol, Itraconazol, Sepraconazol, Clindamycin, Cephalosporine, Chloramphenicol, Erythromycin, 5-Fluoruracil, Etoposid, Fluctytosin, Ganciclovir, Griseofulvin, Gyrasehemmstoffe, Isoniacid, Lincosamide, Mebendazol, Mefloquin, Metronidazol, Nitroimidazole, Novobiocin, Platinverbindungen, Polymyxin B, Praziquantel, Pyrimethamin, Rifamipicin, Saquinavir, Streptomycin, Sulfonamide, Tetracycline, Trimethoprim, Vancomycin, Zidovudin;
- Antipyretika, Analgetika, antiinflammatorische Mittel, Paracetamol, Ibuprofen, Ketoprofen, Oxaprozin, Acetylsalicylsäure, Morhpin, Oxaprozin, Propoxyphen, Phenylbutazon;
- Antibiotika
   Rifampicin, Griseofulvin, Chloramphenicol, Cycloserin, Erythromycin, Penicilline wie Penicillin G, Streptomycin, Tetracyclin;
- Antiepileptika
   Hydantoine, Carbamazepin;
- Antitussiva und Antiasthmatika
   Diphenhydramin;
- Antirheumatika
   Chloroquin, Indomethacin, Goldverbindungen, Phenylbutazon, Oxyphenylbutazon, Penicillinamin;
- Hypnotika
   Barbiturate, Phenobarbital, Zolpidem, Dioxopiperidine,
   Ureide;
- Psychopharmaka, Neuroleptika
   Perazin, Promazin, Sulpirid, Thioridazin, Chlorpromazin, Meprobamat, Triflupromazin, Melperon, Clozapin, Risperdion, Reserpin;
- Tranquillantien;
- Antidepressiva
   Imipramin, Paroxetin, Viloxazin, Moclobemid;
- Psychotonika;
- Psychomimetika;
- Diuretika
   Kaliumcanrenoat, Schleifendiuretika, Furosemid, Hydrochlorothiazid, Spironolacton, Thiazide, Triamteren;
- Hormone
   Androgene, Antiandrogene, Gestagene, Glucocorticoide, Oestrogene, Cortisol, Dexamethason, Prednisolon, Testosteron, Adiuretin, Oxytocin, Somatropin, Insulin;
- Immunsuppresiva
   Ciclosporin;
- Bronchodilatoren;
- Muskelrelaxantien, Tranquillantien
   Carisoprodol, Tetrazepam, Diazepam, Chlordiazepoxid;
- Enzyme
   Lipase, Phytase;
- Gichtmittel
   Allopurinol, Colchicin;
- Antikoagulatien
   Cumarine;
- Antiepileptika
   Phenotoin, Phenobarbital, Primidon, Valproinsäure, Carbamazepin;
- Antihistaminika
   Chlorphenoxamin, Dimenhyrinat;
- Antimimetika;
- Antihyperttonika, Antiarryhythmika
   Lidocain, Procainamid, Chinidin, Calciumanatagonisten, Glyceroltrinitrat, Isosorbiddinitrat, Isosorbid-5-mononitrat, Pentaerythrityltetranitrat, Nifedipine, Diltiazem, Felodipin, Verapamil, Reserpin, Minoxidil, Reserpin, Captopril, Enlanapril, Lisinopril;
- Sympathomimetika
   Norfenefrin, Oxedrin, Midodrin, Phenylephrin, Isoprenalin, Salbutamol, Clenbutorol, Ephedrin, Tyramin, Isoprenalin, β-Blocker wie Alprenolol, Metoprolol, Bisoprolol;
- Antidiabetika
   Biguanide, Sulfonyharnstoffe, Carbutamid, Tolbutamid, Glibenclamid, Metformin, Acarbose, Troglitazon;
- Eisenpräparationen;
- Vitamine und Vitaminoide
   beispielsweise Ascorbinsäure, Tocopherol, Tocopherolacetat, Vitamin A und Vitamin A-Derivate, Vitamin K und Vitamin K-Derivate oder Vitamin D und Vitamin D-Derivate, Riboflavin, Vitamin B₁₂, Nicotinsäure, Nicotinsäureamid, Pyridoxin Hydrochlorid, Biotin, Folsäure, Folsäurederivate wie Tetrahydrofolsäure, 5-Methyl-tetrahydrofolsäure, 10-Formyl-tetrahydrofolsäure oder 5-Formyl-tetrahydrofolsäure;
   Carotinoide, wie z.B. β-Carotin, Lycopin, Lutein, Astaxanthin oder Zeaxanthin;
   Mehrfach ungesättigte Fettsäuren, wie z.B. Linolsäure, Linolensäure, Arachidonsäure, Docohexaensäure oder Eicosapentaensäure;
   Verbindungen mit Vitamin- oder Coenzymcharakter, z.B. Carnitin, Cholinchlorid, Taurin, Kreatin, Ubichinone, S-Methylmethionin oder S-Adenosylmethionin;
- ACE-Hemmer
   Captopril, Ramipril, Enalapril;
- Anabolika;
- Iod-Verbindungen;
- Röntgenkontrastmittel;
- ZNS-aktive Verbindungen;
- Antiparkinsonmittel
   Biperiden, Benzatropin, Amantadin, opioide Analgetika, Barbiturate, Bezodiazepine, Disulfiram, Lithiumsalze, Theophyllin, Valproinat, Neuroleptika;
- Zytostatika;
- Antispasmolytika;
- Vasodilatoren
   Naftidrofuryl, Pentoxifyllin.

Es können auch Zubereitungen der biologisch aktiven Stoffe in Form "fester Lösungen" erhalten werden. Der Begriff "feste Lösungen" ist dem Fachmann geläufig (s. Chiou und Riegelman, J. Pharm. Sci. 60, 1281-1302 (1971)). In festen Lösungen von pharmazeutischen Wirkstoffen in Polymeren oder anderen Matrices liegt der Wirkstoff molekulardispers in der Matrix vor.

Als Matrixkomponenten eignen sich prinzipiell alle thermoplastisch verarbeitbaren Polymere, die eine pH-abhängige Wasserlöslichkeit aufweisen. Insbesondere sind dies Polymere mit Säure (z.B. Carboxylate)- oder Basen (z.B. Amine) Funktionalitäten oder mit säure- oder baseninstabilen Funktionalitäten (z.B. Ester).

Als Polymere kommen beispielsweise die Homo- und -Copolymere des N-Vinylpyrrolidons in Betracht, wobei Vinylacetat bevorzugtes Comonomer ist.

Geeignete Polymere sind beispielsweise auch Copolymere aus Vinylacetat und Crotonsäure wie z.B. Luviset® CA 66, BASF, ein Copolymer aus 90 Gew.-% Vinylacetat und 10 Gew.-% Crotonsäure. Ebenso eignen sich Copolymere aus Methacrylsäure und Ethylacrylat wie z.B. Kollicoat® MAE 100 P, BASF, oder Luvimer® 100 P (Terpolymer aus 67 Gew.-% t-Butylacrylat, 23 Gew.-% Methacrylsäure und 10 Gew.-% Ethylacrylat) der BASF, weiterhin auch nicht- oder teilhydrolisierte Polyvinylacetate, wie z.B. die Vinnapas-Marken (bis zu 100% Vinylacetat) der Firma Wacker, und beispielsweise Dimethylaminoethylacrylatcopolymere.

Bevorzugt werden Polymere eingesetzt, die als Comonomere radikalisch polymerisierbare monoolefinisch ungesättigte Carbonsäuren, wie z.B. Acrylsäure, Methacrylsäure oder Crotonsäure enthalten.

Als zusätzliche Comonomere werden bevorzugt monoolefinisch ungesättigte Carbonsäureester mit bis zu 6 Kohlenstoffatomen, wie z.B. Methacrylsäuremethyl-, -ethyl-, n-butyl- oder -tert-butylester, Acrylsäuremethyl-, -ethyl-, n-butyl oder -tert-butylester, Vinylacetat und Vinylpropionat verwendet.

Besonders bevorzugt sind Polymere, die mindestens 30 Gew.-% Acrylsäure oder Methacrylsäure als Comonomer enthalten. Zudem kommen auch Terpolymere mit z.B. Dimethylaminoethylacrylat und Derivaten, Butylestern oder Acrylamiden in Betracht.

Besonders bevorzugt werden biologisch aktive Substanzen und Polymere enthaltende Matrices, welche gewünschtenfalls zusätzlich Weichmacher und weitere Formulierungshilfsmittel enthalten, die sich bei einem pH-Wert von > 5 in Wasser oder wässrigen Systemen lösen oder dispergieren, sodaß eine feste Darreichungsform der jeweiligen Formulierung die Aktivsubstanz genügend schnell und vollständig in den Magen-Darm-Trakt von Tier oder Mensch freisetzt. Bei pH-Werten von < 3 sollten diese Formulierungen nicht oder deutlich schlechter in Wasser oder wässrigen Systemen löslich oder dispergierbar sein. Dies ist beispielsweise bei der Verwendung von hydrophoben Aktivsubstanzen wie Vitamin-A mit Matrixpolymeren aus Acrylsäure oder Methacrylsäure und Acrylestern (Methacryl- und Acrylester) mit einem Acryl- oder Methacrylsäureanteil von > = 30 % der Fall. Die Freisetzungsgeschwindigkeit hängt allerdings in jedem Fall von der verwendeten Polymermatrix, den optional verwendeten Weichmachern und anderen Additiven, als auch von der Aktivsubstanz ab.

Neben Polymer und Wirkstoff können derartige Formulierungen übliche extrusionstechnische und formulierungsrelevante Zusätze, wie z.B. Weichmacher und Stabilisatoren enthalten.

Als Weichmacher werden z.B. Polyethylenglykole, Triacetin, Triethylcitrat oder Propandiol verwendet.

Stabilisatoren, Tenside etc. und extrusionstechnische Hilfsstoffe

Je nach Anwendungsgebiet und Verarbeitbarkeit können die Anteile an Wirkstoff, Polymer und Additiven in weiten Grenzen variieren. Die einzige Randbedingungen sind die thermoplastische Verarbeitbarkeit und die beschriebenen Löslichkeitseigenschaften der Formulierung. Im allgemeinen wird der Wirkstoffgehalt in einem Bereich von 5 bis 90, vorzugsweise 5 bis 80, besonders bevorzugt 5 bis 60 Gew.-% liegen. Den Rest bilden Polymere, im allgemeinen von 10 bis 55 Gew.-%, sowie Formulierungshilfsstoffe.

Mit Hilfe des erfindungsgemäßen Verfahrens ist es möglich, wirkstoffhaltige Formulierungen durch Schmelzextrusion und Unterwassergranulierung in einem unlöslichen pH-Bereich herzustellen und diese nach Abtrennung in einem löslichen pH-Bereich wieder aufzulösen (Unterwassergranulierung von pH-löslichkeitsabhängigen, wirkstoffhaltigen Schmelzen). Die Fertigung mit Wasser als Kühlmedium ist preiswert, umweltschonend, sicher und einfach. Es ist möglich dem Wasser lösliche Additive wie z.B. Salze hinzuzufügen, um die Löslichkeit der Formulierung zu vermindern.

So ist es beispielsweise möglich, eine polymere, thermoplastisch verarbeitbare Matrix aus Methacrylsäure und Ethylacrylat bei pH-Werten von 3-4 Unterwasser zu granulieren und die hergestellten Pellets nach der Abtrennung in Wasser bei pH-Werten von 7-8 aufzulösen. Die Lösungseigenschaften der Gsamtrezeptur werden durch die verwendeten Wirkstoffe, Weichmacher und Additive mitbestimmt.

Die erfindungsgemäßen Granulate eignen sich insbesondere zur Verwendung in Futtermitteln, Nahrungsergänzungsmitteln oder dietätischen Mitteln, weiterhin für die Herstellung von Arzneimitteln für den Human- und Veterinärbereich.

Ebenso eignen die Granulate sich für Waschmittelformulierungen, die als biologisch aktive Substanzen Enzyme oder Aromastoffe enthalten.

Die Granulate können als solche eingesetzt werden. Weiterhin eignen sie sich als Kapselfüllungen oder zur Verpressung zu Tabletten.

### Beispiele

### Allgemeine Herstellvorschrift:

Alle festen Einsatzstoffe wurden entsprechend der Rezeptur gemischt und über eine Differentialdosierwaage in die Förderzone eines gleichlaufenden, dichtkämmenden Doppelschneckenextruders ZSK 30 (Firma Werner & Pfleiderer GmbH, Stuttgart, Deutschland) bei Raumtemperatur eingefahren. Nach einer weiteren Förderzone (80°C) folgen 2 Aufheizzonen (110°C) und anschließend 4 Misch- und Knetzonen (120°C) an die ein seitliches Pumpaggregat angebracht ist, über das die entsprechenden Flüssigkomponenten eingespeist werden. Nach zwei weiteren Zonen (120°C) wurde die homogenisierte Schmelze über eine Zahnradpumpe (140°C) in eine Unterwassergranulieranlage des Typs Laborgranulierung-Gala: Lab Pelletizing System LPS (9 - ca. 50 kg/h) (GALA Kunststoff- Kautschukmaschinen GmbH, Xanten, Deutschland) eingespeist. Über das Anfahrventil wurde die Schmelze (140°C) durch eine Lochplatte 30 x 1.0 mm mit rotierender Messerwalze in einen geschlossenen Prozeßwasserkreislauf (25°C) eingefahren, wobei der pH-Wert des Prozeßwassers mit 1 molarer Schwefelsäure auf pH 3 eingestellt war. Die so konfektionierten Pellets wurden über eine Umwälzpumpe in einem Zentrifugaltrockner vom Prozeßwasser getrennt. Das Prozeßwasser wird je nach verwendeter Matrix vorher mit Säuren oder Basen auf den entsprechenden pH-Wert eingestellt.

### Beispiel 1

### Feste Einsatzstoffe:

Kollicoat MAE 100 P (Methacrylsäure/Ethylacrylat-Copolymer, 50 Gew.-% MAS, 50 Gew.-% EA; BASF AG), Pulver Lutrol( E 6000 (Polyethylenglykol, MG 6000 BASF AG), Pulver Flüssige Komponente:
Vitamin-A-Öl: Mischung aus Vitamin-A-acetat, 2.8 Mio IE/G unstab. (80 Gew.-%) (BASF AG) und Ethoxyquin (20 Gew.-%) (Raluquin®, Raschig AG) wurde bei 60°C über ein beheiztes Pumpenaggregat zugefahren.

Eine physikalische Mischung aus 65 Gew.-Teilen Kollicoat MAE 100 P und 15 Gew.-Teilen Lutrol E 6000 wurde wie in der allgemeinen Durchführung beschrieben in den Extruder dosiert, gefördert, homogenisiert und aufgeschmolzen. Durch eine seitliche Dosierzone wurden 20 Gew.-Teile Vitamin-A-Öl zugefahren und eingearbeitet. Die Schmelze wurde anschließend in oben beschriebener Weise durch Unterwassergranulation zu sphärischen 1 mm Pellets verarbeitet.

### Dispergierung:

1.0 g der durch Unterwassergranulation hergestellten Pellets wurden unter Rühren (Magnetrührwerk Ikamag RET-G, Janke & Kunkel GmbH & Co.KG., Staufen, Germany; Teflonrührer 1 = 4 cm) in ein zylindrisches 1 l Glasgefäß mit einem Durchmesser von 10 cm mit 1000 ml dest. Wasser (T = 20°C) bei einer Rührgeschwindigkeit von 300 U/min eingetragen (0.1% ige Dispersion). Das destillierte Wasser wurde im ersten Fall zuvor mit 0.1 M HCl auf pH 3 eingestellt (Probe 1) und im zweiten Fall mit 0.1 M NaOH auf pH 8 eingestellt (Probe 2). Nach 20 min wurde über 50 µm Siebe abfiltriert und der Siebrückstand nach dem Trocknen (Vakuumtrockenschrank, 40°C, 4h) in Gew.-%, bezogen auf die Ausgangsmenge (1.0 g) ermittelt.

| Beispiel 1 | Siebrückstand [Gew.-%] |
|---|---|
| Probe 1 | 98 |
| Probe 2 | 0,3 |

Die konfektionierte Rezeptur aus Beispiel 1 ließ sich ohne Substanzverlust bei pH 3 Unterwasser granulieren. Sie dispergierte innerhalb von 20 min nahezu vollständig in Wasser mit dem pH-Wert 8, was eine Resorption im Darm ermöglichen würde.

### Beispiel 2

### Feste Einsatzstoffe:

Luvimer 100 P (t-Butylacrylat/Methacrylsäure/Ethylacrylat-Terpolymer), BASF AG

### Flüssige Komponente:

Vitamin-A-Öl: Mischung aus Vitamin-A-acetat, 2.8 Mio IE/G unstab. (80 Gew.-%) (BASF AG) und Ethoxyquin (20 Gew.-%) (Raluquin, Raschig AG) wurden bei 60°C über ein beheiztes Pumpenaggregat zugefahren.

80 Gew.-Teile Luvimer 100 P wurden wie in der allgemeinen Durchführung beschrieben in den Extruder dosiert, gefördert und aufgeschmolzen. Durch eine seitliche Dosierzone wurden 20 Gew.-Teile Vitamin-A-Öl zugefahren und eingearbeitet. Die Schmelze wurde anschließend in oben beschriebener Weise durch Unterwassergranulation zu sphärischen, beigefarbenen, leicht trüben aber homogenen Pellets mit einem Durchmesser von 1 mm verarbeitet.

### Beispiel 3

### Feste Einsatzstoffe:

Ascorbinsäure, Vitamin C (kristalline Ware), BASF AG Luviset CA 66 (Crotonsäure/Vinylacetat-Copolymer), BASF AG

### Flüssigkomponente:

Triacetin (Triacetylpropantriol), Fluka Chemie AG, Schweiz

Eine physikalische Mischung aus 60 Gew.-Teilen Luviset CA 66 und 30 Gew.-Teilen Vitamin C wurde wie in der allgemeinen Durchführung beschrieben in den Extruder dosiert, homogenisiert und gefördert. Durch eine seitliche Dosierzone wurden 10 Teile Triacetin zugefahren und eingearbeitet. Die Schmelze wurde anschließend in oben beschriebener Weise durch Unterwassergranulation zu sphärischen 1 mm Pellets verarbeitet.

Die so hergestellten Pellets (1g pro Liter Wasser) waren bei pH 8 in Wasser innerhalb von 2 Stunden klar löslich. Reines Luviset CA 66 - Perlpolymerisat löste sich unter diesen Bedingungen deutlich langsamer (> 8 Stunden).

### Beispiel 4

Eine physikalische Mischung aus 69 Gew.-Teilen Kollicoat MAE 100 P, 30 Gew.-Teilen Ibuprofen und 1 Gew.-Teil hochdisperse Kieselsäure (Aerosil 200) wurde wie in der allgemeinen Vorschrift beschrieben in den Extruder dosiert, gefördert, homogenisiert und aufgeschmolzen. Die Schmelze wurde anschließend durch Unterwassergranulation zu spärischen Pellets mit einem mittleren Durchmesser von 1 mm verarbeitet.

## Patentansprüche

1. Verfahren zur Herstellung von Granulaten, die biologisch aktive Substanzen enthalten, welche für die Herstellung von Arzneimitteln, Futtermitteln, Nahrungsergänzungsmitteln und dietätischen Mitteln verwendet werden, in denen die biologisch aktiven Substanzen homogen dispergiert in einer Matrix auf Basis mindestens eines thermoplastisch verarbeitbaren Polymeren, welches in wässrigem Medium eine pH-Wert abhängige Löslichkeit aufweist, vorliegen, durch homogenes Vermischen der Einsatzstoffe in der Schmelze und anschliessender Extrusion und Formgebung, **dadurch gekennzeichnet, daß**, die Formgebung in einem Kühlmedium erfolgt, in dem die Granulate nicht löslich oder dispergierbar sind, wobei als Kühlmedium Wasser eingesetzt wird, dessen pH-Wert so eingestellt ist, daß die Granulate sich nicht lösen oder dispergierbar sind.

2. Verfahren nach Anspruch 1, **dadurch gekennzeichnet, daß** die thermoplastisch verarbeitbaren Polymere saure oder basische Gruppen oder in Säuren oder Basen instabile Gruppen enthalten.

3. Verfahren nach einem der Ansprüche 1 oder 2, **dadurch gekennzeichnet, daß** Polymere eingesetzt werden, die als Comonomer monoolefinisch ungesättigte Carbonsäuren enthalten.

4. Verfahren nach einem der Ansprüche 1 bis 3, **dadurch gekennzeichnet, daß** die Polymeren eines oder mehrere Comonomere, ausgewählt aus der Gruppe bestehend aus Acrylsäure, Methacrylsäure und Crotonsäure, enthalten.

5. Verfahren nach einem der Ansprüche 1 bis 4, **dadurch gekennzeichnet, daß** die Polymere als Comonomere monoolefinisch ungesättigte Carbonsäureester enthalten.

6. Verfahren nach einem der Ansprüche 1 bis 5, **dadurch gekennzeichnet, daß** die Polymere eines oder mehrere Comonomere, ausgewählt aus der Gruppe bestehend aus Acrylsäuremethylester, Acrylsäureethylester, Acrylsäurebutylester, Methacrylsäuremethylester, Methacrylsäureethylester, Methacrylsäurebutylester, Vinylacetat und Vinylpropionat, enthalten.

7. Verfahren nach einem der Ansprüche 1 bis 6, daß die Polymere als Comonomere mindestens 30 Gew.-% Acrylsäure oder Methacrylsäure enthalten.

8. Verfahren nach einem der Ansprüche 1 bis 7, **dadurch gekennzeichnet, daß** zusätzlich Formulierungshilfsmittel eingearbeitet werden.

## Claims

1. A process for producing pellets comprising biologically active substances which are used for producing drugs, feedstuff, food supplements and dietetic compositions, in which the biologically active substances are homogeneously dispersed in a matrix based on at least one thermoplastic polymer, which polymer has in aqueous medium a pH-dependent solubility, by homogeneous mixing of the starting materials in the melt and subsequent extrusion and shaping, which comprises the shaping being performed in a cooling medium in which the pellets are not soluble or dispersible, the cooling medium used being water set to a pH such that the pellets do not dissolve or are not dispersible.

2. A process as claimed in claim 1, wherein the thermoplastic polymers comprise acidic or basic groups or groups which are unstable in acids or bases.

3. A process as claimed in either of claims 1 and 2, wherein polymers are used which comprise monoolefinically unsaturated carboxylic acids as comonomer.

4. A process as claimed in one of claims 1 to 3, wherein the polymers comprise one or more comonomers selected from the group consisting of acrylic acid, methacrylic acid and crotonic acid.

5. A process as claimed in one of claims 1 to 4, wherein the polymers comprise monoolefinically unsaturated carboxylic esters as comonomers.

6. A process as claimed in one of claims 1 to 5, wherein the polymers comprise one or more comonomers selected from the group consisting of methyl acrylate, ethyl acrylate, butyl acrylate, methyl methacrylate, ethyl methacrylate, butyl methacrylate, vinyl acetate and vinyl propionate.

7. A process as claimed in one of claims 1 to 6, wherein the polymers comprise at least 30% by weight of acrylic acid or methacrylic acid as comonomer.

8. A process as claimed in one of claims 1 to 7, wherein formulation aids are additionally incorporated.

## Revendications

1. Procédé de préparation de granules qui contiennent des substances biologiquement actives qui sont utilisées pour la préparation de médicaments, de fourrages, de compléments alimentaires et de produits diététiques dans lesquels les substances biologiquement actives se présentent dispersées de manière homogène dans une matrice à base d'au moins un polymère que l'on peut traiter de manière thermoplastique et qui présente en milieu aqueux une solubilité qui est fonction de la valeur du pH, par mélange homogène des matières mises en oeuvre dans la masse fondue et ensuite extrusion et mise en forme, **caractérisé en ce que** la mise en forme a lieu dans un milieu réfrigérant dans lequel les granules ne sont pas solubles ou dispersables, de l'eau, dont la valeur du pH est ajustée de façon que les granules ne se dissolvent pas ou ne puissent pas se disperser, étant introduite comme milieu réfrigérant.

2. Procédé suivant la revendication 1, **caractérisé en ce que** les polymères que l'on peut traiter de manière thermoplastique contiennent des groupes acides ou basiques ou des groupes instables dans des acides ou des bases.

3. Procédé suivant l'une des revendications 1 et 2, **caractérisé en ce qu'**on met en oeuvre des polymères qui contiennent, comme comonomère, des acides carboxyliques monooléfiniquement insaturés.

4. Procédé suivant l'une des revendications 1 à 3, **caractérisé en ce que** les polymères contiennent un ou plusieurs comonomères choisis parmi le groupe constitué de l'acide acrylique, de l'acide méthacrylique et de l'acide crotonique.

5. Procédé suivant l'une des revendications 1 à 4, **caractérisé en ce que**, comme comonomères, les polymères contiennent des esters d'acide carboxylique monooléfiniquement insaturés.

6. Procédé suivant l'une des revendications 1 à 5, **caractérisé en ce que** les polymères contiennent un ou plusieurs comonomères choisis parmi le groupe constitué d'acrylate de méthyle, d'acrylate d'éthyle, d'acrylate de butyle, de méthacrylate de méthyle, de méthacrylate d'éthyle, de méthacrylate de butyle, d'acétate de vinyle et de propionate de vinyle.

7. Procédé suivant l'une des revendications 1 à 6, **caractérisé en ce que**, comme comonomères, les polymères contiennent au moins 30% en poids d'acide acrylique ou d'acide méthacrylique.

8. Procédé suivant l'une des revendications 1 à 7, **caractérisé en ce qu'**en supplément des adjuvants de formulation sont incorporés.
